# EUROPEAN PATENT SPECIFICATION

(11) **EP 3 877 386 B1**
(45) Date of publication and mention of the grant of the patent: **29.06.2022**
(21) Application number: 19845755.8
(22) Date of filing: 06.11.2019
(51) Int. Cl.: C07D 451/10

(54) **SCOPOLAMINE PRODUCTION**
HERSTELLUNG VON SCOPOLAMIN
PRODUCTION DE SCOPOLAMINE

(30) Priority: 07.11.2018 ZA 201807451
(43) Date of publication of application: 15.09.2021
(73) Proprietor: The University of Johannesburg, Johannesburg 2008 (ZA)
(72) Inventor: JIYANE, Pangaman, 2024 Randburg (ZA); NDINTEH, Derek Tantoh, 2194 Windsor (ZA)
(74) Representative: Ponti & Partners, S.L.P
(86) International application number: PCT/ZA2019/050069
(87) International publication number: WO 2020/097635

(56) References cited:
- CN-A- 108 164 523
- CN-A- 108 610 339
- FR-A1- 2 405 947

## Description

### Technical field of the invention

This invention relates to the production of Scopolamine. More particularly it relates to the production of Scopolamine by means of extraction from plant material.

### Background to the invention

Scopolamine, also known as Hyoscine, is a well-known drug used inter alia for motion sickness and nausea. It is also a precursor to a number of other drugs. Scopolamine is expensive to synthesize and it is therefore economically viable to be extracted from plants of the Solanaceae family.

CN108164523 A discloses a process for extracting scopolamine from belladonna by using macroporous adsorption technology to separate and purify scopolamine.

CN108610339 A discloses a technology for extraction and separation of scopolamine from low content datura metel. This technology includes: adopting ultrasonic-assisted dichloromethane process for extraction, then using acid water and carbon tetrachloride for extraction to realize preliminary enrichment of scopolamine, and then combining macroporous resin with crystallization technology for purification to obtain high purity scopolamine hydrobromide.

FR2405947 discloses the preparation of scopolamine and hyoscyamine hydrobromides by cold extraction of alkaloids with alkaline alcohol, concentration of extract after acidification, extraction of concentrate with CHCl₃, distillation of CHCl₃ and treatment of the residue with HBr in alcohol.

Previously, the applicant invented an extraction and purification method of scopolamine in flos daturae. The method included the following steps: firstly obtaining extract liquid of flos daturae; filtering the extract liquid by means of a ceramic membrane, concentrating by means of a nanofiltration membrane, recovering ethanol, and obtaining a mixture; dissolving the mixture by using diluted hydrochloric acid, carrying out suction filtering, and obtaining filtrate; extracting the filtrate, and reserving the aqueous solution; adjusting the pH value by using a water phase, re-extracting four times, and collecting a chloroformic solution; drying the chloroformic solution, recovering chloroform, and obtaining total alkaloids; and separating the total alkaloids by using alkaline aluminum oxide column chromatography, recovering chloroform, and obtaining scopolamine.

The applicant wishes to improve the yield and the scalability of the above method.

It is an object of the invention to provide a simplified method to produce Scopolamine which is well suited for scale up to process 45 000kg or more of plant material per year.

### General description of the invention

According to the invention there is provided a method for the production of Scopolamine, which method includes the steps of:
grinding plant material which contains Scopolamine;
adding the grinded plant material to a Sulfuric acid solution, preferably 1 %, and allowing to stand in the solution for 1 to 4, preferably 2 to 3 days;
filtration separation of the acid solution from the plant material;
repeating the previous steps with the residue plant material and combining the acidic filtrates;
neutralising with sodium bicarbonate to a pH of between 7.5 and 9.5, preferably between 8 and 9;
addition, preferably immediate, of dichloromethane for the extraction of the alkaloids in the neutralised solution to the organic layer and allowing to stand for 1 to 4 days, preferably 2 to 3 days;
separation of the bottom organic layer from the upper aqueous layer;
evaporation of the dichloromethane solvent to obtain the coloured scopolamine rich extract;
adding the scopolamine rich extract to dichloromethane solvent and mixing with a NaOH, preferably 1%, solution and separating the layers;
evaporation of the dichloromethane solvent to obtain the less coloured scopolamine rich extract;
optionally, adding the less coloured scopolamine rich extract to dichloromethane solvent and mixing with a sodium bicarbonate, preferably 1 %, solution and separating the layers; and
evaporation of the dichloromethane solvent to obtain the final scopolamine rich extract.

The second last optional step improves the purity of the final product.

The plant material may preferably be the seeds of Flos Daturae. It is to be appreciated that the seeds have less chlorophyll, which follows the extraction process to some extent.

The extraction method described is significantly less complex than known methods, does not need special equipment like columns and separation membranes, uses only one low cost solvent and produces a high yield with high purity. The method can also be economically scaled up as required to process 45 tons or more of plant material per year. The extraction method is also tuned to focus on scopolamine from a mixture of alkaloids contained in Flos Daturae.

### Detailed description of the invention

The invention is now described by way of example.

500 Grams of Flos Daturae seeds were grinded and added to 1 500 ml of a 1% Sulfuric acid solution and allowed to stand in the solution for 2 days. The mixture was filtered and 700 ml of the acid solution was recovered. This step can preferably be repeated to increase the yield. The filtrate was neutralised with sodium bicarbonate to a pH of 9. A 1000 ml of dichloromethane was mixed with the filtrate and allowed to stand for 2 days. The bottom organic layer was separated from the upper aqueous layer and the dichloromethane solvent was evaporated using a roto evaporator to obtain the coloured scopolamine rich extract. Once off, adding the scopolamine rich extract to 1000ml dichloromethane solvent and mixing with a 1% NaOH 1000ml, solution and separating the layers. The dichloromethane solvent was again removed in a roto evaporator to obtain the less coloured scopolamine rich extract. The less coloured scopolamine rich extract was added to 1000ml dichloromethane solvent and mixed with a 1000ml 1% sodium bicarbonate solution and the layers were separated. This step is also once off. The dichloromethane solvent was evaporated to obtain 12 grams of the final scopolamine rich extract, which contains 70% scopolamine in a pharmaceutically acceptable mixture.

The use of sulphuric acid in the first step removes fat soluble impurities. The long extraction time of 2 days improves the yield significantly and it appears that the conversion from organic acid to salt is relatively slow. The selective neutralisation to pH of 8 to 9 avoided neutralising other compounds and their subsequent extraction to yield a purer product. Again, longer than normal extraction time improved the yield and the further steps improved the purity compared to known extraction methods.

It shall be understood that the example is provided for illustrating the invention further and to assist a person skilled in the art with understanding the invention and is not meant to be construed as unduly limiting the reasonable scope of the invention.

## Claims

1. A method for the production of Scopolamine, which method includes the steps of:
grinding plant material which contains Scopolamine;
adding the grinded plant material to a Sulfuric acid solution and allowing to stand in the solution for 1 to 4 days,
filtration separation of the acid solution from the plant material;
repeating the previous steps with the residue plant material and combining the acidic filtrates;
neutralising with a alkaline solution to a pH of between 7.5 and 9.5;
addition of dichloromethane for the extraction of the alkaloids in the neutralised solution to the organic layer and allowing to stand for 1 to 4 days;
separation of the bottom organic layer from the upper aqueous layer;
evaporation of the dichloromethane solvent to obtain the coloured scopolamine rich extract;
adding the scopolamine rich extract to dichloromethane solvent and mixing with a NaOH solution and separating the layers; and
evaporation of the dichloromethane solvent to obtain the less coloured scopolamine rich extract.

2. A method for the production of Scopolamine as claimed in Claim 1, wherein the Sulfuric acid solution is 1 %.

3. A method for the production of Scopolamine as claimed in Claim 1 or Claim 2, wherein the Sulfuric acid solution is allowed to stand for 2 to 3 days.

4. A method for the production of Scopolamine as claimed in any one of the previous claims, wherein the alkaline solution is a sodium bicarbonate solution at a pH of between 8 and 9.

5. A method for the production of Scopolamine as claimed in any one of the previous claims, wherein the Dichloromethane is added immediately.

6. A method for the production of Scopolamine as claimed in any one of the previous claims, wherein the organic layer is allowed to stand for 2 to 3 days.

7. A method for the production of Scopolamine as claimed in any one of the previous claims, wherein the NaOH solution is 1%.

8. A method for the production of Scopolamine as claimed in any one of the previous claims, wherein the less coloured scopolamine rich extract is added to dichloromethane solvent and mixed with a 1% sodium bicarbonate solution and the layers separated and the dichloromethane solvent evaporated to obtain the final scopolamine rich extract.

## Patentansprüche

1. Verfahren zur Herstellung von Scopolamin, wobei das Verfahren die folgenden Schritte umfasst:
Zermahlen von Pflanzenmaterial, das Scopolamin enthält;
Zugabe des zermahlenen Pflanzenmaterials zu einer Schwefelsäurelösung und 1 bis 4 Tage langes Stehenlassen in der Lösung,
Filtration der Säurelösung aus dem Pflanzenmaterial;
Wiederholung der vorhergehenden Schritte mit der Restmenge des Pflanzenmaterials und Kombinieren der sauren Filtrate;
Neutralisation mit einer alkalischen Lösung auf einen pH Wert zwischen 7,5 und 9,5;
Zugabe von Dichlormethan zur organischen Schicht zwecks Extraktion der Alkaloide in der neutralisierten Lösung und 1 bis 4 Tage langes Stehenlassen;
Trennung der unteren organischen Schicht von der oberen wässrigen Schicht;
Verdampfen des Dichlormethan-Lösungsmittels, um den farbigen Scopolamin-reichen Extrakt zu erhalten;
Zugabe des Scopolamin-reichen Extrakts zum Dichlormethan-Lösungsmittel und Mischen mit einer NaOH-Lösung sowie Trennung der Schichten; und
Verdampfen des Dichlormethan-Lösungsmittels, um weniger farbigen Scopolamin-reichen Extrakt zu erhalten.

2. Verfahren zur Herstellung von Scopolamin gemäß Anspruch 1, wobei die Schwefelsäurelösung 1% beträgt.

3. Verfahren zur Herstellung von Scopolamin gemäß Anspruch 1 oder Anspruch 2, wobei man die Schwefelsäurelösung 2 bis 3 Tage lang stehen lässt.

4. Verfahren zur Herstellung von Scopolamin gemäß einem der vorhergehenden Ansprüche, wobei es sich bei der alkalischen Lösung um eine Natriumbicarbonat-Lösung bei einem pH-Wert zwischen 8 und 9 handelt.

5. Verfahren zur Herstellung von Scopolamin gemäß einem der vorhergehenden Ansprüche, wobei das Dichlormethan sofort zugegeben wird.

6. Verfahren zur Herstellung von Scopolamin gemäß einem der vorhergehenden Ansprüche, wobei die organische Schicht 2 bis 3 Tage lang stehen gelassen wird.

7. Verfahren zur Herstellung von Scopolamin gemäß einem der vorhergehenden Ansprüche, wobei die NaOH Lösung 1% beträgt.

8. Verfahren zur Herstellung von Scopolamin gemäß einem der vorhergehenden Ansprüche, wobei der weniger farbige Scopolamin-reiche Extrakt zum Dichlormethan-Lösungsmittel gegeben und mit einer 1%igen Natriumbicarbonat-Lösung gemischt wird, die Schichten getrennt werden und das Dichlormethan-Lösungsmittel verdampft wird, um den endgültigen Scopolamin-reichen Extrakt zu erhalten.

## Revendications

1. Procédé de production de scopolamine, lequel procédé comprend les étapes suivantes:
broyage de matière végétale qui contient de la scopolamine;
ajout de la matière végétale broyée à une solution d'acide sulfurique et la laisser reposer dans la solution pendant 1 à 4 jours;
filtration de la solution acide à partir de la matière végétale;
répétition des étapes précédentes avec la matière végétale résiduelle et combinaison des filtrats acides;
neutralisation avec une solution alcaline à un pH compris entre 7,5 et 9,5;
ajout de dichlorométhane à la couche organique pour l'extraction des alcaloïdes dans la solution neutralisée; laisser reposer pendant 1 à 4 jours;
séparation de la couche organique inférieure de la couche aqueuse supérieure;
évaporation du solvant de dichlorométhane pour obtenir l'extrait riche en scopolamine coloré;
ajout de l'extrait riche en scopolamine au solvant de dichlorométhane et son mélange avec une solution de NaOH et séparation des couches; et
évaporation du solvant de dichlorométhane pour obtenir l'extrait riche en scopolamine moins coloré.

2. Procédé de production de scopolamine selon la revendication 1, dans lequel la solution d'acide sulfurique est de 1%.

3. Procédé de production de scopolamine selon la revendication 1 ou la revendication 2, dans lequel on laisse reposer la solution d'acide sulfurique pendant 2 à 3 jours.

4. Procédé de production de scopolamine selon l'une quelconque des revendications précédentes, dans lequel la solution alcaline est une solution de bicarbonate de sodium à un pH compris entre 8 et 9.

5. Procédé de production de scopolamine selon l'une quelconque des revendications précédentes, dans lequel le dichlorométhane est ajouté immédiatement.

6. Procédé de production de scopolamine selon l'une quelconque des revendications précédentes, dans lequel on laisse reposer la couche organique pendant 2 à 3 jours.

7. Procédé de production de scopolamine selon l'une quelconque des revendications précédentes, dans lequel la solution de NaOH est de 1%.

8. Procédé de production de scopolamine selon l'une quelconque des revendications précédentes, dans lequel l'extrait riche en scopolamine moins coloré est ajouté au solvant de dichlorométhane et mélangé avec une solution de bicarbonate de sodium à 1%, les couches étant séparées et le solvant de dichlorométhane étant évaporé pour obtenir l'extrait riche en scopolamine final.
